# EUROPÄISCHE PATENTANMELDUNG

(11) **EP 0 760 377 A2**
(43) Veröffentlichungstag der Anmeldung: **05.03.1997**
(21) Anmeldenummer: 96113283.4
(22) Anmeldetag: 20.08.1996
(51) Int. Cl.: C07K 14/805, C07K 17/02, G01N 33/52, G01N 33/72

(54) **Verfahren zum Stabilisieren des Gehalts glykierten Proteins einer Probe auf einem Matrixmaterial**

(30) Priorität: 24.08.1995 DE 19531173
(71) Anmelder: BOEHRINGER MANNHEIM GMBH, 68298 Mannheim (DE)
(72) Erfinder: Nagel, Rolf, 68642 Bürstadt (DE); Mistele, Jürgen, 68809 Neulussheim (DE)

(57) **Zusammenfassung**

Gegenstand der Erfindung ist ein Verfahren zum Stabilisieren des Gehalts glylierten Proteins einer Probe auf einem Matrixmaterial, dadurch gekennzeichnet, daß das Matrixmaterial mit Borsäurepuffer, pH größer oder gleich 10,5 oder einem Übergangsmetallsalz imprägniert ist sowie ein entsprechendes Matrixmaterial, ein Element zum Sammeln, Transportieren und Lagern von zu analysierendem Probenmaterial mit einem solchen Matrixmaterial und ein System enthaltend ein solches Element und eine verschließbare Umhüllung.

## Beschreibung

Die Erfindung betrifft ein Verfahren zum Stabilisieren des Gehalts glykierten Proteins einer Probe auf einem Matrixmaterial sowie ein hierfür entsprechend behandeltes Matrixmaterial. Die Erfindung betrifft weiterhin ein Element zum Sammeln, Transportieren und Lagern von zu analysierendem Probenmaterial enthaltend ein saugfähiges Matrixmaterial und ein System enthaltend ein solches Element und eine verschließbare Umhüllung, in der das Element transportiert werden kann.

Bei Patienten mit Diabetes mellitus ist die Glykierung von Hämoglobin und Serumproteinen erhöht. Die Erhöhung ist abhängig von der Glukosekonzentration und der Inkubationszeit des Proteins mit Glukose. Die Glykierung von Serumproteinen, einschließlich Hämoglobin erfolgt in diesen Fällen nicht enzymatisch, sondern unkatalysiert durch chemische Reaktion der Glukose mit Aminogruppen der Proteine. Die Fachwelt geht davon aus, daß die Konzentration eines bestimmten Protein-Glukose-Addukts sowohl die Glukosekonzentration über eine bestimmte Zeit als auch die Umsatzrate des Proteins widerspiegelt. Glykiertes Hämoglobin wird als Indikator der mittleren Blutglukosekonzentration während der letzten zwei bis drei Monate vor Blutabnahme und Untersuchung angesehen. Glykiertes Serumprotein gibt die Blutglukosekonzentration während einer kürzeren Zeitspanne wieder. Die Bestimmung von glykiertem Protein, wie glykiertem Hämoglobin (HbA_{1c}) oder glykiertem Serumprotein hat deshalb eine erhebliche Bedeutung für die langfristige glycämische Kontrolle von Diabetespatienten.

Für eine Untersuchung von Blut auf den Gehalt an glykiertem Protein muß die Probe oft zu einem weiter entferntem Labor transportiert werden. Während dieser Transportzeit und einer sich eventuell noch anschließenden Wartezeit darf sich der Gehalt an glykiertem Protein in der Probe möglichst nicht verändern. In Clinical Chemistry 29, 1080 - 1082 (1983) wird über die Untersuchung von Blutproben auf glykiertes Hämoglobin berichtet, die längere Zeit gelagert wurden. Es ergibt sich hieraus, daß Vollblut bis zu 21 Tagen bei Raumtemperatur gelagert werden kann, ohne daß sich der HbA_{1c}-Gehalt wesentlich verändert.

Der Transport flüssiger Blutproben ist jedoch aufwendig und mit Risiken, wie zum Beispiel Bruch des Transportgefäßes verbunden. Außerdem ist für die Entnahme von Vollblut oft eine Venenpunktion notwendig, obwohl für die Untersuchung an sich auch so kleine Probenmengen ausreichen würden, wie sie bei der Abnahme von Kapillarblut aus der Fingerbeere erhalten werden. Für den Transport und die Untersuchung kleinerer Probenmengen wurden deshalb Methoden entwickelt, bei denen Kapillarblut auf Filterpapier aufgegeben und dort eintrocknen gelassen wird. Das Filterpapier wird anschließend zum Ort der Untersuchung transportiert. Dort wird aus dem Filterpapier eine Scheibe mit Probe ausgestanzt, eluiert und das Eluat untersucht. Auf eine solche Methode bezieht sich der Bericht in Clinical Chemistry 28, 386 - 387 (1982). In diesem Bericht wird festgestellt, daß sich bei der Blutprobenlagerung auf Filterpapier der Gehalt an glykiertem Protein gegenüber der ursprünglichen Probe stark verändert. Nach Lagerung von Vollblut auf Filterpapier werden deutlich erhöhte Meßwerte für glykiertes Protein gefunden.

In Clinical Chemistry 32, 869 - 871 (1986) wird die Imprägnierung von Filterpapier mit Glukoseoxidase zur Verhinderung der durch Lagerung von Blut auf Filterpapier verursachten Erhöhung des Gehaltes an glykierten Hämoglobin beschrieben. Allerdings vermag die Imprägnierung mit Glukoseoxidase die Zunahme an glykiertem Protein nicht vollständig zu verhindern. Die falsche Werterhöhung kann durch diese Maßnahme lediglich vermindert werden. Ein weiterer Nachteil der Imprägnierung mit Glukoseoxidase ist deren eigene Instabilität während der Lagerung unter üblichen Lagerbedingungen.

Ähnliches ergibt sich aus einem Artikel in Diabetes Care 10, 352 - 355 (1987). Dort wird berichtet, daß die Behandlung von Filterpapier mit Glukoseoxidase oder auch mit Ethanol eine falsche Werterhöhung für glykiertes Hämoglobin bei Lagerung von Blut auf Filterpapier nicht zufriedenstellend verhindern kann.

Die Aufgabe der vorliegenden Erfindung bestand deshalb darin, den Gehalt an glykiertem Protein in einer Probe bei Lagerung auf einem Matrixmaterial zu stabilisieren. Nach Lagerung glykierten Proteins auf einem Matrixmaterial sollte für das glykierte Protein ein Wert gefunden werden, wie er nach der Probenahme und vor Lagerung bestand.

Diese Aufgabe wird durch den in den Patentansprüchen näher charakterisierten Gegenstand gelöst.

Gegenstand der Erfindung ist ein Verfahren zum Stabilisieren des Gehalts glykierten Proteins einer Probe auf einem Matrixmaterial durch Imprägnieren des Matrixmaterials mit einem Borsäurepuffer, pH größer oder gleich 10,5. Eine vergleichbare Stabilisierung ist auch möglich, wenn das Matrixmaterial ein Übergangsmetallsalz trägt.

Gegenstand der Erfindung ist außerdem das Matrixmaterial zur Aufnahme von auf seinen Gehalt an glykiertem Protein zu prüfendem Probenmaterial, welches dadurch gekennzeichnet ist, daß es mit Borsäurepuffer, pH größer oder gleich 10,5 imprägniert ist oder daß es ein Übergangsmetallsalz trägt.

Weiterhin ist Gegenstand der Erfindung ein Element zum Sammeln, Transportieren und Lagern von zu analysierendem Probenmaterial, enthaltend ein saugfähiges Matrixmaterial, wobei das Element dadurch gekennzeichnet ist, daß das Matrixmaterial mit Borsäurepuffeer pH größer oder gleich 10,5 imprägniert ist oder ein Übergangsmetallsalz trägt.

Schließlich ist Gegenstand der Erfindung ein System, enthaltend ein wie vorstehend charakterisiertes Element und eine verschließbare Umhüllung, in der das Element transportiert werden kann, dadurch gekennzeichnet, daß das Element ein solches ist, wie es vorstehend bereits als erfindungsgemäß gekennzeichnet worden ist.

Als Matrixmaterial werden im Rahmen der vorliegenden Erfindung saugfähige Materialien bezeichnet, die in der Lage sind, Flüssigkeit aufzunehmen, die glykiertes Protein enthält. Glykiertes Protein, also Zuckerreste tragendes Protein, befindet sich vor allem in Blut, aber auch in von Blut abgeleiteten Probenmaterialien wie Serum oder Plasma und darüberhinaus auch in anderen Körperflüssigkeiten, wie beispielsweise Urin oder Speichel. Materialien, die solche Probenmaterialien aufsaugen können, sind bevorzugt faserig, können grundsätzlich aber auch nicht-faserig sein. Bevorzugte faserige saugfähige Matrixmaterialien sind Vliese, Gewebe oder Gewirke. Vliese sind ganz besonders bevorzugt. Die faserigen Matrixmaterialien können Glas-, Zellulose-, Polyesterfasern, aber auch Viskose und Polyvinylalkohol enthalten. Vliesmaterialien, enthaltend schmelzbare Copolyesterfasern neben Glasfasern, Polyesterfasern, Polyamidfasern, Zellulosefasern oder Zellulosederivatefasern, wie sie in der europäischen Patentanmeldung 0 571 941 beschrieben sind, können ebenfalls vorteilhaft als Matrixmaterial eingesetzt werden. Nicht-faserige Materialien können beispielsweise Membranen sein.

Erfindungsgemäß hat es sich gezeigt, daß Probenmaterial enthaltend glykiertes Protein, das sich auf einem Matrixmaterial befindet, sehr gut ohne wesentliche Veränderung des Gehalts an glykiertem Protein gelagert werden kann, wenn das Matrixmaterial mit Borsäurepuffer, pH größer oder gleich 10,5 imprägniert ist oder wenn das Matrixmaterial ein Übergangsmetallsalz trägt. Die Konzentration des Borsäurepuffers ist hierbei von eher untergeordneter Bedeutung. Besonders gute Ergebnisse werden erzielt, wenn der Borsäurepuffer einen pH-Wert größer oder gleich 11 besitzt. Geeignete Pufferkonzentrationen liegen im Bereich zwischen 300 und 1000 mmol / l, das entspricht etwa 18,6 - 62 g / 100 ml.

Eine analoge gut stabilisierende Wirkung üben Übergangsmetallsalze, wie beispielsweise Nickel- oder Kupfersalze aus. Nickelsalze sind besonders bevorzugt. Wasserlösliche Übergangsmetallsalze werden erfindungsgemäß bevorzugt eingesetzt. Gut geeignet sind beispielsweise entsprechende Chloride. Um erfindungsgemäß wirksam zu sein haben sich erfindungsgemäß Übergangmetallsalzkonzentrationen auf dem Matrixmaterial von größer 5 g / m², besonders bevorzugt größer 10 g / m² als geeignet erwiesen.

Probenmaterial enthaltend glykiertes Protein, wie beispielsweise glykiertes Hämoglobin, das auf ein wie vorstehend beschriebenes Matrixmaterial aufgegeben worden war, hat auch bei Messung nach längerer Lagerung bei erhöhter Temperatur Werte für das glykierte Protein ergeben, die mit der ursprünglichen Konzentration vergleichbar sind. Als Probenmaterial kommen alle Flüssigkeiten in Frage, die vorstehend bereits genannt worden sind. Blut oder von Blut abgeleitete Proben, wie Plasma oder Serum sind ganz besonders bevorzugt. Grundsätzlich können aber erfindungsgemäß alle Flüssigkeiten eingesetzt werden, die glykierte Proteine enthalten können.

Als glykierte Proteine kommen ebenfalls grundsätzlich alle Proteine in Frage, die durch chemische Reaktion eines Proteins mit Glukose entstanden sind. Ganz besondere Bedeutung hat glykiertes Hämoglobin, das auch als HbA_{1c} bezeichnet wird. Glykiertes Albumin oder glykierte Serumproteine sind ebenfalls in diesem Zusammenhang zu nennen.

Ein wie vorstehend beschriebenes Matrixmaterial, das erfindungsgemäß zum Stabilisieren des Gehalts glykierten Proteins einer Probe verwendet werden kann, kann auch in einem Element zum Sammeln, Transportieren und Lagern von zu analysierendem Probenmaterial enthalten sein. Ein solches Element kann zum Beispiel analog dem von Boehringer Mannheim vertriebenen HbA_{1c} Via Post^{®}beschaffen sein. Dieses Element ist in Klin. Lab. 39, 1080-1082 (1993) beschrieben. Statt eines runden, unimprägnierten Filterpapierausschnitts, der auf einem Trägermaterial befestigt ist, kann erfindungsgemäß ein vorstehend beschriebenes behandeltes Matrixmaterial eingesetzt werden, auf das flüssiges Probenmaterial, speziell Blut, so aufgegeben werden kann, daß es vom Matrixmaterial aufgesaugt wird. So kann das Element zum Ort der Untersuchung transportiert werden, wo aus dem Matrixmaterial die Probe eluiert und das Eluat untersucht werden kann. Durch Verwendung des erfindungsgemäßen Matrixmaterials kann das darin enthaltende Probenmaterial längere Zeit gelagert werden, ohne daß sich der Gehalt an glykiertem Protein wesentlich verändert. Insbesondere für den interessanten Parameter HbA_{1c} hat sich der Einsatz des erfindungsgemäßen Matrixmaterials als besonders geeignet erwiesen.

Zur Anwendung der vorliegenden Erfindung hat sich als besonders geeignet ein Element erwiesen, das eine erste und eine zweite Schicht saugfähigen Matrixmaterials so sich berührend nebeneinander auf einem inerten Trägermaterial in einem einen Flüssigkeitsübertritt ermöglichenden Kontakt angeordnet enthält, daß Flüssigkeit von der ersten in die zweite Schicht gelangen kann, wenn die erste Schicht mit Flüssigkeit gefüllt ist und die erste Schicht nach Aufbringen und Eintrocknen des Probenmaterials vollständig von der zweiten Schicht abgetrennt werden kann. Ein solches Element ist in der deutschen Patentanmeldung P 19 523 061.2 beschrieben. Erfindungsgemäß wird für die erste Schicht ein Matrixmaterial verwendet, das wie eingangs beschrieben imprägniert ist und entweder einen Borsäurepuffer mit pH größer oder gleich 10,5 oder ein Übergangsmetallsalz trägt.

Als eine wichtige Eigenschaft der in einem solchen Element einsetzbaren saugfähigen Matrixmaterialien ist deren Saugfähigkeit zu beachten. Die Saugfähigkeit des Matrixmaterials der ersten Schicht soll gleich groß oder größer sein als die der benachbarten Schicht. Dadurch wird vermieden, daß störende Saugeffekte ausgebildet werden, wenn Probenmaterial auf die erste Schicht aufgebracht wird.

Die Saugfähigkeit kann nach DIN 53106 bestimmt werden. Hierzu werden Proben von 200 +/-1 mm Länge und 15 +/- 0,1 mm Breite lotrecht mit ihrem unteren Ende 25 mm in destilliertem Wasser eingetaucht und der Weg, den das Wasser in 10 min zurücklegt, in mm gemessen. Wie unterschiedliche Saugfähigkeiten bei Matrixmaterialien mit gleichen Bestandteilen eingestellt werden können, ist dem Fachmann bekannt. Beispielsweise können bei der Herstellung von Vliesen unterschiedliche Dicken verwendet werden. Je dicker die eingesetzten Fasern sind, desto geringer ist die Saugfähigkeit. Ein weiterer Weg ist die Variation der Dichte von Vliesen. Durch eine Erhöhung der Dichte wird die Saugfähigkeit reduziert.

Bei der Verwendung von Geweben haben Gewebe mit feineren Fasern eine größere Saugfähigkeit als Gewebe mit gröberen Fasern. Aber auch über unterschiedliche Arten der Verzwirnung der Fäden kann die Saugfähigkeit gesteuert werden. Ebenso können über die Webarten Unterschiede in der Saugfähigkeit erzielt werden. Weitere Variationsmöglichkeiten der Saugfähigkeit können durch die Verwendung von unterschiedlichen Fasermischungen erreicht werden. So wird zum Beispiel durch den Zusatz von hydrophoben Fasern die Saugfähigkeit verringert.

Als inertes Trägermaterial, auf dem die Matrixmaterialschichten sich befinden, kommen insbesondere steife Materialien in Frage, wie beispielsweise Kunststoffolien, Karton, beschichtetes Papier usw. Auf dem inerten Trägermaterial sind die Matrixmaterialschichten so befestigt, daß die Flüssigkeitsaufnahme durch die Matrixmateralien nicht beeinträchtigt wird. Dies ist durch Verwendung eines doppelseitig klebenden Bandes oder beispielsweise auch durch die Verwendung von Schmelzkleber möglich.

Die Matrixmaterialschichten müssen auf dem inerten Trägermaterial so befestigt sein, daß die erste Schicht nach Aufbringen und Eintrocknen flüssigen Probenmaterials vollständig von der zweiten Schicht abgetrennt werden kann. Dies ist insbesondere dann möglich, wenn die erste Schicht nur relativ lose oder nur punktuell befestigt ist.

Die beiden Matrixmaterialschichten müssen so nebeneinander sich berührend auf dem Trägermaterial angeordnet sein, daß ein Flüssigkeitsübertritt von der ersten Schicht in die zweite Schicht möglich ist, wenn die erste Schicht mit Flüssigkeit gefüllt ist. Dies ist dann möglich, wenn zumindest eine Kantenberührung der beiden Schichten vorliegt. Noch besser ist jedoch eine leichte Überlappung der beiden Schichten. Besonders bevorzugt sind die Schichten dann so angeordnet, daß die zweite Schicht die erste Schicht leicht überlappt.

Die Größe der Matrixmaterialschichten muß so gewählt werden, daß die erste Schicht, die später auch als Auswerteschicht verwendet wird, mit der Probenflüssigkeit vollständig gefüllt werden kann. Überschüssige Probenflüssigkeit wird dann von der zweiten Schicht aufgenommen. Welche Probenmengen ausreichend sind zur Bestimmung eines bestimmten Analyten hängt von der Art des zu bestimmenden Analyten ab. In der Regel reichen jedoch 5-20 µl, meisten 10 µl Probe aus. Dieses Volumen muß von der ersten Matrixschicht aufgenommen und später wieder eluiert werden können. Zur Sicherheit sollte die zweite Matrixschicht, die die Funktion einer Saugschicht darstellt, ein größeres Volumen aufnehmen können. Saugvolumen von 10 - 50, bevorzugt 10 - 30 µl, besonders bevorzugt 20 µl, reichen hierzu in der Regel aus. Zweckmäßigerweise sollten die üblichen Abmessungen der Matrixmaterialschichten so beschaffen sein, daß das Saugvolumen der beiden Matrixmaterialschichten zusammengenommen mindestens 30 µl, besser mindestens 50 µl, beträgt. Über eine solche Dimensionierung wird gewährleistet, daß sowohl mit kleinen als auch mit großen Flüssigkeitstropfen auf die erste Matrixschicht verschiedene erfindungsgemäße Elemente die gleiche Menge an Probe aufgebracht wird. Die kleinere erste Schicht hat zur Erreichung eines ausreichenden Saugvolumens in der Regel eine Fläche von 3x3 bis 8x8 mm.

Durch die vorbeschriebene Anordnung der Matrixmaterialschichten wird eine homogene Verteilung flüssigen Probenmaterials in der ersten Schicht erreicht. Dadurch, daß die erste Schicht vollständig mit flüssigem Probenmaterial gefüllt wird, können so nach Abtrennung und Eluierung der ersten Schicht immer reproduzierbare Mengen zur Untersuchung gelangen.

Zum Transport eines erfindungsgemäßen Elementes nach Aufgabe des Probenmaterials zur Analysestation hat es sich als zweckmäßig erwiesen, dieses innerhalb einer verschließbaren Umhüllung auf den Weg zu bringen. Umhüllung und Element bilden so ein System. Als verschließbare Umhüllung eignet sich beispielsweise ein Umschlag, wie ein Briefumschlag, der ein Vorderteil und zwei Seitenteile sowie eine Rückenklappe und eine Verschlußlasche beinhaltet, mit der der Umschlag beispielsweise verklebt werden kann. Eine solche Umhüllung ist ebenfalls in der deutschen Patentanmeldung P 19 523 061.2 beschrieben.

Die Erfindung wird in den nachfolgenden Beispielen weiter erläutert.

### Beispiel 1

### Stabilisierung von HbA1c durch Borsäurepuffer

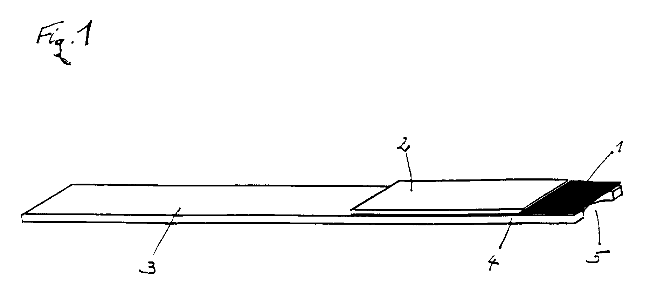

Auf eine Polyesterfolie (3) der Abmessungen 49 x 6 mm mit einer halbkreisförmigen Ausstanzung (5) von 5 mm an einem kurzseitigen Ende wird mit Hilfe eines doppelseitigen Klebebandes (4) eine erste Schicht (1) saugfähigen Matrixmaterials, wie in Figur 1 dargestellt, so auf der Trägerfolie fixiert, daß sie mit einer Breite von 0,5 bis 1 mm auf das Klebeband (4) geklebt wird. Über diese relativ schmale Fixierung wird die spätere Abnehmbarkeit positiv beeinflußt. Die zweite Schicht (2) saugfähigen Matrixmaterials wird auf einer Breite von 5 mm oder mehr verklebt.

Für die erste Schicht saugfähigen Matrixmaterials wird ein Vlies, das auf einer Papiermaschine hergestellt wurde und folgende Daten aufweist, verwendet:

80 Teile Polyesterfasern (Faserdurchmesser 1,7 Dtex), 20 Teile Viskose, 20 Teile Polyvinylalkohol; Flächengewicht 80 g / m² ; Saughöhe 102 mm (DIN 53106).

Dieses Vlies wurde mit einem der in Tabelle 1 aufgeführten Borsäurepuffer (62 g Borsäure in 800 ml destilliertem Wasser suspendiert, mit 5 Mol / l Kaliumhydroxidlösung aufgewünschten pH-Wert eingestellt und mit destilliertem Wasser auf 1000 ml aufgefüllt) imprägniert, bei 50°C getrocknet und anschließend auf die Größe 6 x 6 mm zugeschnitten. Diese Matrix nimmt eine Flüssigkeitsmenge von ca. 10 µl auf

Für die zweite Schicht saugfähigen Matrixmaterials wird ein Vlies eingesetzt, das der ersten Schicht entspricht, aber nicht mit Borsäurepuffer imprägniert ist.

Auf so gefertigte erfindungsgemäße Elemente werden jeweils ca. 10 µl EDTA-Blut, enthaltend 5,1% HbA_{1c}, mit 500 mg / dl Glukose aufgestockt, appliziert und bei Raumtemperatur mindestens 2 Stunden getrocknet. Zur Simulation eines Transportes wurde der getrocknete Probenträger in Versandkuverts bei 35 ° C für 5 Tage belastet.

Nach Entfernung der ersten Matrixschicht mit einer Pinzette wird das Matrixmaterial in 1 ml Hämolysereagenz für den Tina-quant®-Test von Boehringer Mannheim GmbH (Deutschland) (Bestellnummer 1 488 457) eluiert. Anschließend wird HbA_{1c} bestimmt nach der immunologischen Bestimmungsmethode von Boehringer Mannheim GmbH (Deutschland) auf einem Hitachi 717-Gerät von Boehringer Mannheim GmbH mit Reagenz der Bestellnummer 1 488 414 von Boehringer Mannheim GmbH.

In Tabelle 1 sind die Meßergebnisse für Elemente zusammengefaßt, bei denen das Matrixmaterial keinen Borsäurepuffer enthielt oder Borsäurepuffer verschiedenen pH-Wertes.

**Tabelle 1**

| | **Lagerbedingungen 5 Tage bei** | |
|---|---|---|
| **Matrix imprägniert mit** | **2 bis 8° C HbA**_{**1c**} **(%)** | **35 ° C HbA**_{**1c**} **(%)** |
| **ohne Borsäure** | **5,6** | **11,2** |
| **Borsäure pH 6,3** | **5,3** | **10,6** |
| **Borsäure pH 7,0** | **5,4** | **10,1** |
| **Borsäure pH 8,0** | **5,3** | **9,1** |
| **Borsäure pH 9,0** | **5,2** | **7,9** |
| **Borsäure pH 10,0** | **5,5** | **7,0** |
| **Borsäure pH 10,5** | **5,0** | **6,0** |
| **Borsäure pH 11,0** | **5,2** | **5,5** |
| **Borsäure pH 12,0** | **5,1** | **5,3** |

Im Ergebnis erkennt man, daß Borsäurepuffer ab einem pH-Wert von 10,5 zu einer solchen Stabilisierung des nicht-enzymatisch glykosylierten Proteins führen, daß auch nach Temperaturbelastung ausreichend unveränderte Konzentrationswerte vorliegen.

### Beispiel 2

### Stabilisierung von HbA_{1c} durch Nickel(II)chlorid

Analog Beispiel 1 wurden Elemente zum Sammeln, Transportieren und Lagern von zu analysierendem Probenmaterial hergestellt, bei denen jedoch die erste Matrixschicht mit einer NiCl₂-Konzentrationsreihe zwischen 0 und 200 mmol / l NiCl₂ (0; 9,6; 14,4; 19,2; 24,0 und 48 g / l Nickelchlorid-6-Hydrat in 1000 ml destilliertem Wasser gelöst) so getränkt wurde, daß unter-schiedliche Nickelsalzkonzentrationen, wie sie in Tabelle 2 angegeben sind, in der Matrix vorlagen. Als Blutprobe wurde eine solche wie in Beispiel 1 mit 5,1% HbA_{1c}, aufgestockt mit 500 mg / dl Glukose, verwendet. Bestimmung von HbA_{1c} wie in Beispiel 1 ergab die in Tabelle 2 aufgeführten Konzentrationswerte.

**Tabelle 2**

| | **Lagerbedingungen 5 Tage bei** | |
|---|---|---|
| **Matrix imprägniert mit NiCl**_{**2**} **(g/m**^{**2**}**)** | **2 bis 8 ° C HbA**_{**1c**} **(%)** | **35 ° C HbA**_{**1c**} **(%)** |
| **0** | **5,6** | **11,2** |
| **4.2** | **5,4** | **8,2** |
| **6.3** | **5,1** | **6,3** |
| **8.4** | **5,2** | **5,7** |
| **10.5** | **4,9** | **5,8** |
| **21.0** | **5,3** | **4,9** |

## Patentansprüche

1. Verfahren zum Stabilisieren des Gehalts glykierten Proteins einer Probe auf einem Matrixmaterial, dadurch gekennzeichnet, daß das Matrixmaterial mit Borsäurepuffer, pH größer oder gleich 10,5 oder einem Übergangsmetallsalz imprägniert ist.

2. Matrixmaterial zur Aufnahme von auf seinen Gehalt an glykiertem Protein zu prüfendem Probenmaterial, dadurch gekennzeichnet, daß das Matrixmaterial mit Borsäurepuffer, pH größer oder gleich 10,5 oder einem Übergangsmetallsalz imprägniert ist.

3. Matrixmaterial gemäß Patentanspruch 2, dadurch gekennzeichnet, daß das Matrixmaterial mit Borsäurepuffer, pH größer oder gleich 11, imprägniert ist.

4. Matrixmaterial gemäß Patentanspruch 2, dadurch gekennzeichnet, daß das Matrixmaterial ein Nickel- oder Kupfersalz trägt.

5. Matrixmateerial gemäß Patentanspruch 4, dadurch gekennzeichnet, daß das Matrixmaterial ein wasserlösliches Nickelsalz trägt.

6. Element zum Sammeln, Transportieren und Lagern von zu analysierendem Probenmaterial, enthaltend ein saugfähiges Matrixmaterial, dadurch gekennzeichnet, daß das Matrixmaterial ein solches gemäß einem der Patentansprüche 2-5 ist.

7. Element gemäß Patentanspruch 6, dadurch gekennzeichnet, daß das Element eine erste und eine zweite Schicht saugfähigen Matrixmaterials so sich berührend nebeneinander auf einem inerten Trägermaterial in einem einen Flüssikeitsübertrittt ermöglichenden Kontakt angeordnet enthält, daß Flüssigkeit von der ersten in die zweite Schicht gelangen kann, wenn die erste Schicht mit Flüssigkeit gefüllt ist und die erste Schicht nach Aufbringen und Eintrocknen des Probenmaterials vollständig von der zweiten Schicht abgetrennt werden kann, wobei die erste Schicht aus einem Matrixmaterial gemäß einem der Patentansprüche 2-5 besteht.

8. System enthaltend ein Element zum Sammeln, Transportieren und Lagern von zu analysierendem Probenmaterial, mit einem saugfähigen Matrixmaterial und eine verschließbare Umhüllung, in der das Element transportiert werden kann, dadurch gekennzeichnet, daß das Element ein solches gemäß einem der Patentansprüche 6 oder 7 ist.
